(19) Europäisches Patentamt
European Patent Office
Office européen des brevets

(11) Numéro de publication: **0 448 427 B1**

(12) **FASCICULE DE BREVET EUROPEEN**

(45) Date de publication de fascicule du brevet: **11.01.95**

(51) Int. Cl.[6]: **C07C 255/31**, A01N 53/00

(21) Numéro de dépôt: **91400475.9**

(22) Date de dépôt: **21.02.91**

Le dossier contient des informations techniques présentées postérieurement au dépôt de la demande et ne figurant pas dans le présent fascicule.

# (54) Nouveaux esters dérivés d'acide 3-[2-cyano 2-halogéno éthényl], 2,2-diméthyl cyclopropanecarboxylique,leur procédé de préparation et leur application comme pesticides.

(30) Priorité: **27.02.90 FR 9002406**

(43) Date de publication de la demande: **25.09.91 Bulletin 91/39**

(45) Mention de la délivrance du brevet: **11.01.95 Bulletin 95/02**

(84) Etats contractants désignés: **CH DE FR GB IT LI NL**

(56) Documents cités: **EP-A- 0 133 406**

(73) Titulaire: **ROUSSEL-UCLAF
35, Boulevard des Invalides
F-75007 Paris (FR)**

(72) Inventeur: **Babin, Didier
22, rue de la Grenouillette
F-78180 Montigny (FR)**
Inventeur: **Benoit, Marc
Le Cannet Est-Pont de l'Etoile
F-13360 Roquevaire (FR)**
Inventeur: **Demoute, Jean-Pierre
65, Avenue Foch
F-93360 Neuilly Plaisance (FR)**
Inventeur: **Tessier, Jean
30, rue Jean Moulin
F-94300 Vincennes (FR)**

(74) Mandataire: **Tonnellier, Marie-José et al
111, route de Noisy
B.P. no 9
F-93230 Romainville (FR)**

EP 0 448 427 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux esters dérivés d'acide 3-[2-cyano 2-halogéno éthényl] 2,2-diméthyl cyclopropanecarboxylique, leur procédé de préparation et leur application comme pesticides.

On connaissait des esters polyfluorés benzylique de l'acide 3-(2-cyano 2-fluoroéthényle) 2,2-diméthylcyclopropane carboxylique doués de propriétés insecticides cf EP-A 133406.

L'invention a pour objet sous toutes leurs formes stéréoisomères ou sous forme de mélanges de ces stéréoisomères, les composés de formule ($I_A$) :

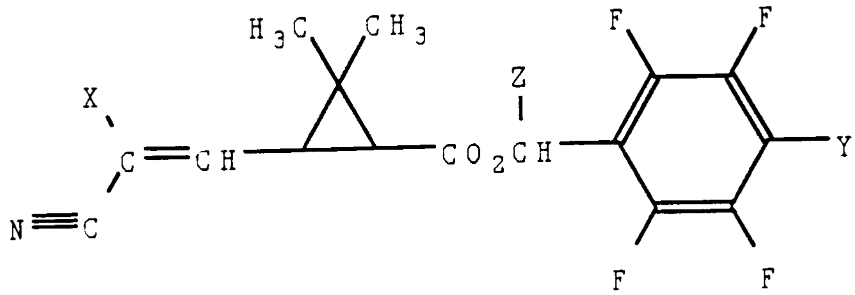

($I_A$)

dans laquelle la copule cyclopropanique est de structure (1R,cis) ou (1R,trans), la géométrie de la double liaison portée par le carbone en 3 pouvant être (E) ou (Z),

- X représente un atome d'halogène,
- Z représente un atome d'hydrogène, un radical $CH_3$, C≡CH ou C≡N,
- Y représente un atome d'hydrogène ou un radical alkyle, alkényle ou alkynyle renfermant jusqu'à 4 atomes de carbone.
- X représente de préférence un atome de fluor, de chlore ou du brome.

Lorsque Y représente un radical alkyle, il s'agit de préférence du radical méthyle, éthyle, propyle, isopropyle, n-butyle ou tertbutyle.

Lorsque Y représente un radical alkyle insaturé, il s'agit d'un radical éthylénique comme par exemple éthényle, propényle ou propadiényle ou d'un radical acétylénique comme par exemple le radical éthynyle ou propynyle.

L'invention a plus particulièrement pour objet les composés de formule ($I_A$) dans lesquels X représente un atome de fluor.

Parmi les composés préférés de l'invention, on peut citer :

- les composés de formule ($I_A$) dans lesquels Z représente un atome d'hydrogène,
- les composés de formule ($I_A$) dans lesquels Y représente un radical alkyle renfermant jusqu'à 4 atomes de carbone et notamment un radical méthyle ou éthyle,
- les composés de formule ($I_A$) dans lesquels Y représente un radical alkényle ou alkynyle renfermant jusqu'à 4 atomes de carbone,
- les composés de formule ($I_A$) dans lesquels Y représente un atome d'hydrogène,
- les composés de formule ($I_A$) dans lesquels la copule cyclopropanique est de structure (1R,cis).
- les composés de formule ($I_A$) dans lesquels la géométrie de la double liaison est (E).

L'invention a tout spécialement pour objet les composés dont la préparation est donnée ci-après dans la partie expérimentale et notamment le produit de l'exemple 1.

L'invention a également pour objet un procédé de préparation des composés de formule ($I_A$) caractérisé en ce que l'on soumet un acide de formule (II) :

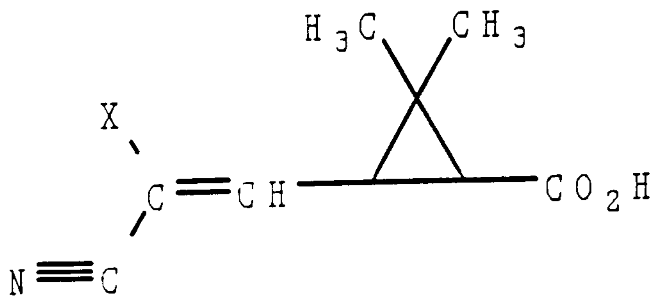

(II)

dans laquelle X conserve sa signification précédente ou un dérivé de cet acide, à l'action d'un alcool de formule (III) :

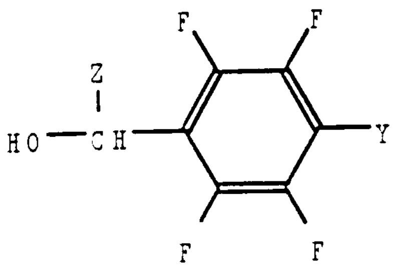

(III)

dans laquelle Y et Z conservent leur signification précédente ou un dérivé fonctionnel de cet alcool pour obtenir le compose de formule (I) correspondant.

Les acides de formule (II) sont des produits connus qui peuvent être préparés comme il est indiqué dans le brevet européen 0133406.

Les alcools de formule (III) sont des composés connus d'une manière générale ; ils peuvent être préparés par exemple selon les procédés décrits dans la demande de brevet européen 0031199, dans les brevets américains 4370346, 4405640, dans le brevet anglais 2171994, dans British Crop Protection Conference Pest and Desease 1986 page 199 ou encore dans la demande de brevet européen 0281439.

Les composés de formule ($I_A$) présentent d'intéressantes propriétés qui permettent leur utilisation dans la lutte contre les parasites. Il peut s'agir par exemple de la lutte contre les parasites des végétaux, qu'il s'agisse des parasites du sol ou des parties aériennes, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule ($I_A$) à la lutte contre les parasites des végétaux, les parasites des locaux et les parasites des animaux à sang chaud.

Les produits de formule ($I_A$) peuvent aussi être utilisés pour lutter contre les insectes et autres parasites du sol, par exemple les coléoptères, comme DIABROTICA, les taupins et les vers blancs, les myriapodes comme les scutigérelles et les blaniules, et les diptères comme les cécydomies et les lépidoptères comme les noctuelles terricoles.

Ils sont utilisés à des doses comprises entre 10 g et 300 g de matière active à l'hectare.

Les produits de formule ($I_A$) peuvent également être utilisés pour lutter contre les insectes dans les locaux, pour lutter notamment contre les mouches, les moustiques et les blattes.

Les produits de formule ($I_A$) sont de plus photostables et sont peu toxiques pour les mammifères.

L'ensemble de ces propriétés fait des produits de formule ($I_A$) des produits qui correspondent parfaitement aux exigences de l'industrie agrochimique moderne : ils permettent de protéger les récoltes tout en préservant l'environnement.

Les produits de formule ($I_A$) peuvent aussi être utilisés pour lutter contre les acariens et les nématodes parasites des végétaux.

Les composés de formule ($I_A$) peuvent encore être utilisés pour lutter contre les acariens parasites des animaux, pour lutter par exemple contre les tiques et notamment les tiques du genre de Boophilus, ceux du

genre Hyalomnia, ceux du genre Amblyomnia et ceux du genre Rhipicéphalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule ($I_A$) définis ci-dessus et notamment l'exemple 1.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensioactif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin), amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2.2.1]hept-5-ène-2,3-dicarboximide, ou le pipéronyl-bis-2-(2'-n-butoxy éthoxy) éthylacétal (ou tropital).

Les composés de formule ($I_A$) présentent une excellente tolérance générale, et l'invention a donc également pour objet les produits de formule ($I_A$), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolone, d'alcool 3,4,5,6-tétrahydrophtalimidométhylique, d'alcool 5-benzyl 3-furyl méthylique, d'alcools 3-phénoxy benzylique et d'alcools alpha-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcools 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les exemples suivants illustrent l'invention sans toutefois la limiter. Les composés des exemples 3, 12, 13, 15 et 16 ne répondent pas à la formule ($I_A$).

**EXEMPLE 1 : [1R-[1alpha,3alpha(E)]]-3-(2-cyano 2-fluoro éthényl) 2,2-diméthyl cyclopropanecarboxylate de (2,3,5,6-tétrafluoro 4-méthyl phényl) méthyle.**

On refroidit à 0°C, une solution renfermant 1,27 g d'acide 3-[2-cyano 2-fluoro éthényl] cyclopropanecarboxylique, 13 $cm^3$ de chlorure de méthylène, 1,36 g de (2,3,5,6-tétrafluoro 4-méthyl phényl) méthanol et 50 mg de 4-(diméthylamino) pyridine. On ajoute goutte à goutte 1,46 g de dicyclohexylcarbodiimide et 5 $cm^3$ de chlorure de méthylène. On agite la suspension obtenue pendant 17 heures à 20°C. On obtient 2,32 g de produit brut que l'on chromatographie sur silice en éluant par le mélange hexane-acétate d'éthyle (9-1). On amène à sec. On obtient 2,19 g de produit recherché Rf = 0,3.

$[alpha]_D$ = + 63,5° ± 2,5° (c = 0,4 % $CHCl_3$) .

En opérant comme à l'exemple 1, à partir des acides et des alcools appropriés, on a obtenu les produit suivants :

**EXEMPLE 2 : [1R-[1alpha,3alpha(E)]]-3-(2-cyano 2-fluoro éthényl) 2,2-diméthyl cyclopropanecarboxylate de [2,3,5,6-tétrafluoro 4-(1,2-propanediényl) phényl] méthyle.**

Rf = 0,3

$[alpha]_D$ = 30° ± 2° (c = 0,45 % $CHCl_3$) .

**EXEMPLE 3 : [1R-[1alpha,3alpha(E)]]-3-(2-cyano 2-fluoro éthényl) 2,2-diméthyl cyclopropanecarboxylate de (2,3,5,6-tétrafluoro 4-méthoxy phényl) méthyle.**

Rf = 0,3

$[alpha]_D$ = 36,5° ± 2° (c = 0,7 % $CHCl_3$ ).

**EXEMPLE 4 : [1R-[1alpha,3alpha(E)]]-3-(2-cyano 2-fluoro éthényl) 2,2-diméthyl cyclopropanecarboxylate de (2,3,5,6-tétrafluoro phényl) méthyle.**

Rf = 0,3

$[alpha]_D$ = 52,5° ± 2,5° (c = 0,6 % $CHCl_3$) .

**EXEMPLE 5 : [1R-[1alpha,3alpha(Z)]]-3-(2-cyano 2-fluoro éthényl) 2,2-diméthyl cyclopropanecarboxylate de (2,3,5,6-tétrafluoro 4-méthyl phényl) méthyle.**

F = 93,6°C
r = 0,2

**EXEMPLE 6 : [1R-[1alpha,3alpha(E)]]-3-(2-cyano 2-fluoro éthényl) 2,2-diméthyl cyclopropanecarboxylate de (2,3,5,6-tétrafluoro 4-éthynyl phényl) méthyle.**

F = 47,5°C
Rf = 0,38

$[alpha]_D$ = + 39° ± 1,5° (c = 1 % $CHCl_3$) .

**EXEMPLE 7 : [1R-[1alpha,3alpha(E)]]-3-(2-cyano 2-fluoro éthényl) 2,2-diméthyl cyclopropanecarboxylate de (2,3,5,6-tétrafluoro 4-éthényl phényl) méthyle.**

**EXEMPLE 8 : [1R-[1alpha,3alpha(Z)]]-3-(2-cyano 2-fluoro éthényl) 2,2-diméthyl cyclopropanecarboxylate de (2,3,5,6-tétrafluoro 4-éthyl phényl) méthyle.**

**EXEMPLE 9 : [1R-[1alpha,3alpha(E)]]-3-(2-cyano 2-fluoro éthényl) 2,2-diméthyl cyclopropanecarboxylate de (2,3,5,6-tétrafluoro 4-éthyl phényl) méthyle.**

**EXEMPLE 10 : [1R-[1alpha,3alpha(E)]]-3-(2-cyano 2-fluoro éthényl) 2,2-diméthyl cyclopropanecarboxylate de (2,3,5,6-tétrafluoro 4-(2-propynyl) phényl) méthyle.**

**EXEMPLE 11 : [1R-[1alpha,3alpha(Z)]]-3-(2-cyano 2-fluoro éthényl) 2,2-diméthyl cyclopropanecarboxylate de (2,3,5,6-tétrafluoro 4-(2-propynyl) phényl) méthyle.**

**EXEMPLE 12 : [1R-[1alpha,3alpha(E)]]-3-(2-cyano 2-fluoro éthényl) 2,2-diméthyl cyclopropanecarboxylate de (2,3,5,6-tétrafluoro 4-(méthoxyméthyl) phényl) méthyle.**

$[alpha]_D$ = + 43° ± 2,5° (c = 0,5% toluène) .

**EXEMPLE 13 : [1R-[1alpha,3alpha(Z)]]-3-(2-cyano 2-fluoro éthényl) 2,2-diméthyl cyclopropanecarboxylate de (2,3,5,6-tétrafluoro 4-(méthoxyméthyl) phényl) méthyle.**

$[alpha]_D$ = - 30° ± 2,5° (c = 0,4% toluène) .

**EXEMPLE 14 : [1R-[1alpha,3alpha(E)]]-3-(2-cyano 2-fluoro éthényl) 2,2-diméthyl cyclopropanecarboxylate de (2,3,5,6-tétrafluoro 4-(2-propényl) phényl) méthyle.**

**EXEMPLE 15 : [1R-[1alpha,3alpha(E)]]-3-(2-cyano 2-fluoro éthényl) 2,2-diméthyl cyclopropanecarboxylate de (pentafluorophényl) méthyle.**

$[alpha]_D$ = + 39° ± 2° (c = 0,8% $CHCl_3$) .

**EXEMPLE 16 : [1R-[1alpha,3alpha(Z)]]-3-(2-cyano 2-fluoro éthényl) 2,2-diméthyl cyclopropanecarboxylate de (pentafluorophényl) méthyle.**

$[alpha]_D$ = - 53° ± 1,5° (c = 1% $CHCl_3$) .

**Exemple 17 : Préparation d'un concentré soluble**

On effectue un mélange homogène de :

| | |
|---|---|
| Produit de l'exemple 1 | 0,25 g |
| Butoxyde pipéronyle | 1,00 g |
| Tween 80 | 0,25 g |
| Topanol A | 0,1 g |
| Eau | 98,4 g |

**Exemple 18** : **Préparation d'un concentré émulsifiable**

On mélange intimement :

| | |
|---|---|
| Produit de l'exemple 2 | 0,015 g |
| Butoxyde de pipéronyle | 0,5 g |
| Topanol A | 0,1 g |
| Tween 80 | 3,5 g |
| Xylène | 95,885 g |

**Exemple 19** : **Préparation d'un concentré émulsifiable**

On effectue un mélange homogène de :

| | |
|---|---|
| Produit de l'exemple 1 | 1,5 g |
| Tween 80 | 20,00 g |
| Topanol A | 0,1 g |
| Xylène | 78,4 g |

**Exemple 20** : **Préparation d'une composition fumigène**

On mélange d'une façon homogène :

| | |
|---|---|
| Produit de l'exemple 1 | 0,25 g |
| Poudre de tabu | 25,00 g |
| Poudre de feuille de cèdre | 40,00 g |
| Poudre de bois de pin | 33,75 g |
| Vert brillant | 0,5 g |
| p-Nitrophénol | 0,5 g |

**ETUDE BIOLOGIOUE**

A. Etude de l'effet d'abattage sur mouche domestique

Les insectes tests sont des mouches domestiques femelles âgées de 4 jours. On opère par pulvérisation directe à la concentration de 0,10 g/l en chambre de Kearns et March en utilisant comme solvant un mélange d'acétone (5 %) et d'Isopar L (solvant pétrolier) (quantité de solvant utilisée 2 $cm^3$ en une seconde). On utilise 50 insectes par traitement. On effectue les contrôles toutes les minutes jusqu'à 10 minutes, puis à 15 minutes et l'on détermine le $KT_{50}$ par les méthodes habituelles.

Les résultats expérimentaux obtenus sont résumés dans le tableau suivant :

| Composés | $KT_{50}$ en mn |
|---|---|
| Exemple 1 | 4,0 |
| Exemple 3 | 3,6 |
| Exemple 4 | 3,6 |
| Exemple 5 | 6,6 |
| Exemple 6 | 3,9 |
| Exemple 7 | 5,7 |
| Exemple 8 | 4,2 |
| Exemple 9 | 6,2 |
| Exemple 10 | 1,3 |
| Exemple 11 | 1,2 |
| Exemple 12 | 4,2 |
| Exemple 13 | 1,7 |

B. Etude de l'effet létal sur larves de Spodoptera Littoralis

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24°C et 65 % d'humidité relative. Après traitement les individus sont placés sur milieu nutritif artificiel (milieu de Poitout).

On effectue le contrôle des mortalités 48 heures après traitement.

Les résultats expérimentaux obtenus sont résumés dans le tableau suivant :

| Composés | $DL_{50}$ en ng/insecte |
|---|---|
| Exemple 1 | 48,3 |
| Exemple 5 | 35,6 |
| Exemple 6 | 69,9 |
| Exemple 7 | 70,8 |
| Exemple 8 | 15,3 |
| Exemple 9 | 51,9 |
| Exemple 10 | 12,9 |
| Exemple 11 | 2,5 |
| Exemple 12 | 54,7 |
| Exemple 13 | 21,6 |

C. Etude de l'activité sur Diabrotica

Les insectes tests sont des larves de dernier stade de Diabrotica.

On traite une rondelle de papier filtre de 9 cm de diamètre, déposée au fond d'une boite de Petri, à l'aide de 2 $cm^3$ de solution acétonique. Après séchage on dépose 10 larves par dose et on effectue le contrôle de mortalité 24 h après le traitement.

On détermine la dose létale 100 ($DL_{100}$) exprimée en mg/litre.

Les résultats obtenus sont les suivants :

| | |
|---|---|
| Produit de l'exemple 1 | 0,6 |
| Produit de l'exemple 3 | 2,5 |
| Produit de l'exemple 4 | 1,25 |
| Produit de l'exemple 6 | 5 |
| Produit de l'exemple 7 | 5 |
| Produit de l'exemple 8 | 5 |
| Produit de l'exemple 9 | 1,25 |
| Produit de l'exemple 10 | 0,6 |
| Produit de l'exemple 11 | 1,25 |

## Revendications

**1.** Sous toutes leurs formes stéréoisomères ou sous forme de mélanges de ces stéréoisomères, les composés de formule ($I_A$) :

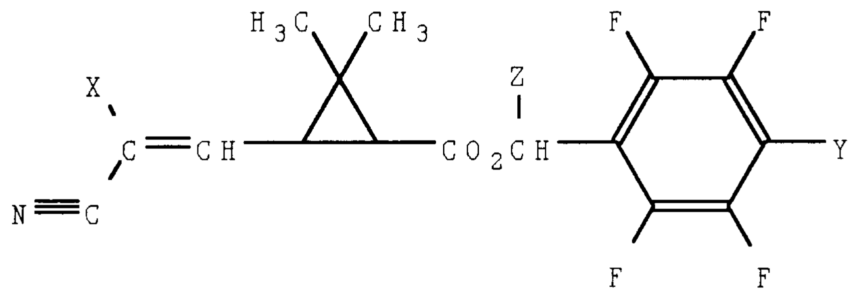

$(I_A)$

dans laquelle la copule cyclopropanique est de structure (1R,cis) ou (1R,trans), la géométrie de la double liaison portée par le carbone en 3 pouvant être (E) ou (Z),

- X représente un atome d'halogène,
- Z représente un atome d'hydrogène, un radical $CH_3$, C≡CH ou C≡N,
- Y représente un atome d'hydrogène ou un radical alkyle, alkényle ou alkynyle renfermant jusqu'à 4 atomes de carbone.

**2.** Les composés de formule $(I_A)$ tels que définis à la revendication 1, dans lesquels X représente un atome de fluor.

**3.** Les composés de formule $(I_A)$ définis à la revendication 1 ou 2, dans lesquels Z représente un atome d'hydrogène.

**4.** Les composés de formule $(I_A)$ définis à l'une quelconque des revendications 1 à 3 dans lesquels Y représente un radical alkyle renfermant jusqu'à 4 atomes de carbone.

**5.** Les composés de formule $(I_A)$ définis à la revendication 4, dans lesquels Y représente un radical méthyle ou éthyle.

**6.** Les composés de formule $(I_A)$ définis à l'une quelconque des revendications 1 à 3, dans lesquels Y représente un radical alkényle ou alkynyle renfermant jusqu'à 4 atomes de carbone.

**7.** Les composés de formule $(I_A)$ définis à l'une quelconque des revendications 1 à 3, dans lesquels Y représente un atome d'hydrogène.

**8.** Les composés de formule $(I_A)$ définis à l'une quelconque des revendications 1 à 7 dans lesquels la copule cyclopropanique est de structure (1R,cis).

**9.** Les composés de formule $(I_A)$ définis à l'une quelconque des revendications 1 à 8, dans lesquels la géométrie de la double liaison est (E).

**10.** Le composé de formule $(I_A)$ défini à la revendication 1, dont le nom suit :

- le [1R-[1alpha,3alpha(E)]]-3-(2-cyano 2-fluoro éthényl) 2,2-diméthyl cyclopropanecarboxylate de (2,3,5,6-tétrafluoro 4-méthyl phényl) méthyle.

**11.** Procédé de préparation des composés de formule $(I_A)$ définis à l'une quelconque des revendications 1 à 10, caractérisé en ce que l'on soumet un acide de formule (II) :

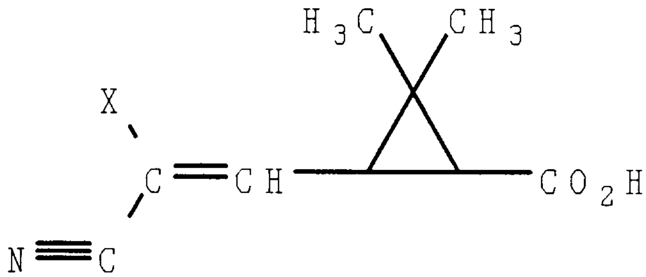

(II)

dans laquelle X conserve sa signification précédente ou un dérivé de cet acide, à l'action d'un alcool de formule (III) :

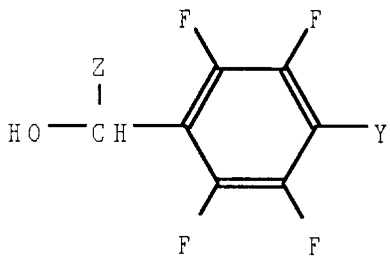

(III)

dans laquelle Y et Z conservent leur signification telle que donnée à la revendication 1 ou un dérivé fonctionnel de cet alcool pour obtenir le composé de formule (I) correspondant.

**12.** Les compositions pesticides renfermant comme principe actif au moins un composé de formule ($I_A$), défini à l'une quelconque des revendications 1 à 9.

**13.** Les compositions pesticides renfermant comme principe actif au moins le composé de formule ($I_A$) défini à la revendication 10.

**14.** Les compositions insecticides renfermant comme principe actif au moins un composé de formule ($I_A$) défini à l'une quelconque des revendications 1 à 10.

**15.** Les compositions pesticides définies à la revendication 12 ou 13 caractérisées en ce qu'elles sont destinées à lutter contre DIABROTICA ou les autres insectes du sol.

## Claims

**1.** In all their stereoisomeric forms or in the form of mixtures of these stereoisomers, the compounds of formula ($I_A$):

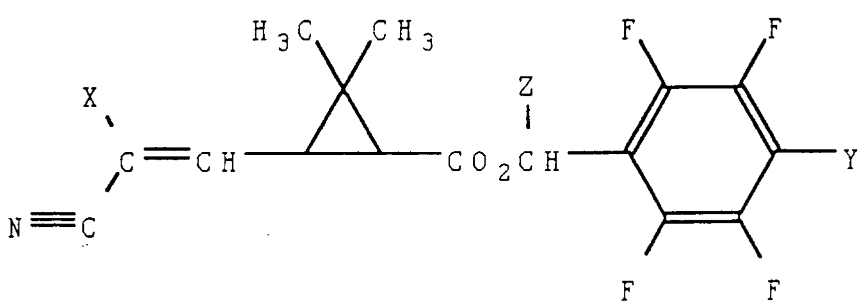

($I_A$)

in which the cyclopropane copula is of (1R,cis) or (1R,trans) structure, the geometry of the double bond carried by the carbon in position 3 being able to be (E) or (Z),

- X represents a halogen atom,
- Z represents a hydrogen atom, a $CH_3$, C≡CH or C≡N radical,
- Y represents a hydrogen atom or an alkyl, alkenyl or alkynyl radical containing up to 4 carbon atoms.

**2.** The compounds of formula ($I_A$) as defined in claim 1, in which X represents a fluorine atom.

**3.** The compounds of formula ($I_A$) defined in claim 1 or 2, in which Z represents a hydrogen atom.

**4.** The compounds of formula ($I_A$) defined in any one of claims 1 to 3 in which Y represents an alkyl radical containing up to 4 carbon atoms.

**5.** The compounds of formula ($I_A$) defined in claim 4, in which Y represents a methyl or ethyl radical.

**6.** The compounds of formula ($I_A$) defined in any one of claims 1 to 3, in which Y represents an alkenyl or alkynyl radical containing up to 4 carbon atoms.

**7.** The compounds of formula ($I_A$) defined in any one of claims 1 to 3, in which Y represents a hydrogen atom.

**8.** The compounds of formula ($I_A$) defined in any one of claims 1 to 7 in which the cyclopropane copula is of (1R,cis) structure.

**9.** The compounds of formula ($I_A$) defined in any one of claims 1 to 8, in which the geometry of the double bond is (E).

**10.** The compound of formula (I) defined in claim 1, the name of which follows:

- (2,3,5,6-tetrafluoro 4-methyl phenyl) methyl [1R-[1alpha, 3alpha(E)]]-3-(2-cyano-2-fluoro ethenyl) 2,2-dimethyl cyclopropanecarboxylate.

**11.** Preparation process for the compounds of formula ($I_A$) defined in any one of claims 1 to 10, characterized in that an acid of formula (II):

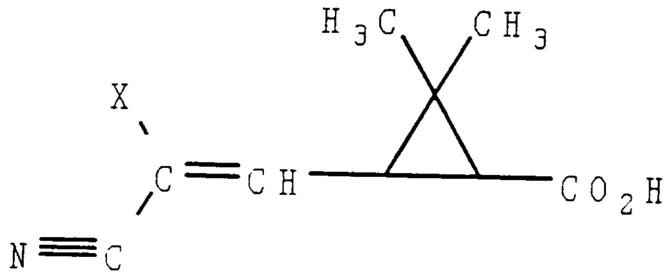

(II)

in which X retains its previous meaning, or a derivative of this acid, is subjected to the action of an alcohol of formula (III):

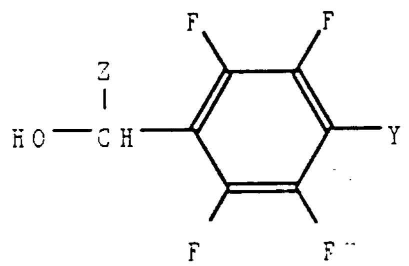

(III)

in which Y and Z retain their previous meaning, or a functional derivative of this alcohol, in order to obtain the corresponding compound of formula (I).

**12.** The pesticide compositions containing as active ingredient at least one compound of formula ($I_A$), defined in any one of claims 1 to 9.

**13.** The pesticide compositions containing as active ingredient at least the compound of formula ($I_A$) defined in claim 10.

**14.** The insecticide compositions containing as active ingredient at least one compound of formula ($I_A$) defined in any one of claims 1 to 10.

**15.** The pesticide compositions defined in claim 12 or 13 characterized in that they are intended to combat DIABROTICA or other insects of the soil.

**Patentansprüche**

**1.** In allen ihren stereoisomeren Formen oder in Form der Mischungen dieser Stereoisomere, die Verbindungen der Formel ($I_A$)

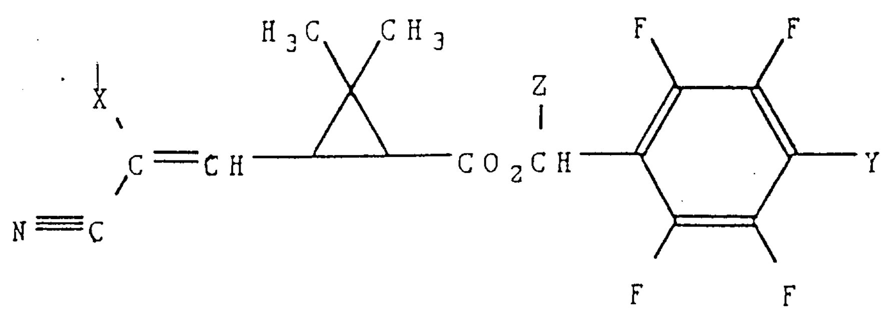

($I_A$)

in der die Cyclopropan-Kupplung die Struktur (1R,cis) oder (1R,trans) besitzt, die Geometrie der durch den Kohlenstoff in 3-Stellung getragenen Doppelbindung (E) oder (Z) sein kann,

- X ein Halogenatom darstellt,
- Z ein Wasserstoffatom, einen Rest $CH_3$, C≡CH oder C≡N bedeutet,
- Y ein Wasserstoffatom oder einen Alkyl-, Alkenyl- oder Alkinylrest mit bis zu 4 Kohlenstoffatomen darstellt.

**2.** Verbindungen der Formel ($I_A$) wie in Anspruch 1 definiert, worin X ein Fluoratom darstellt.

**3.** Verbindungen der Formel ($I_A$) wie in Anspruch 1 oder 2 definiert, worin Z ein Wasserstoffatom darstellt.

**4.** Verbindungen der Formel ($I_A$) wie in irgendeinem der Ansprüche 1 bis 3 definiert, worin Y einen Alkylrest mit bis zu 4 Kohlenstoffatomen darstellt.

**5.** Verbindungen der Formel ($I_A$) wie in Anspruch 4 definiert, worin Y einen Methyl- oder Ethylrest darstellt.

**6.** Verbindungen der Formel ($I_A$) wie in irgendeinem der Ansprüche 1 bis 3 definiert, worin Y einen Alkenyl- oder Alkinylrest mit bis zu 4 Kohlenstoffatomen darstellt.

**7.** Verbindungen der Formel ($I_A$) wie in irgendeinem der Ansprüche 1 bis 3 definiert, worin Y ein Wasserstoffatom darstellt.

**8.** Verbindungen der Formel ($I_A$) wie in irgendeinem der Ansprüche 1 bis 7 definiert, worin die Cyclopropan-Kupplung die Struktur (1R,cis) besitzt.

**9.** Verbindungen der Formel ($I_A$) wie in irgendeinem der Ansprüche 1 bis 8 definiert, worin die Geometrie der Doppelbindung (E) ist.

**10.** Verbindung der Formel ($I_A$) wie in Anspruch 1 definiert, mit der folgenden Bezeichnung:
- [1R-[1α,3α(E)]]-3-(2-Cyano-2-fluorethenyl)-2,2-dimethyl-cyclopropancarbonsäure-(2,3,5,6-tetrafluor-4-methylphenyl)-methylester.

**11.** Verfahren zur Herstellung der Verbindungen der Formel ($I_A$) wie in irgendeinem der Ansprüche 1 bis 10 definiert, dadurch gekennzeichnet, daß man eine Säure der Formel (II)

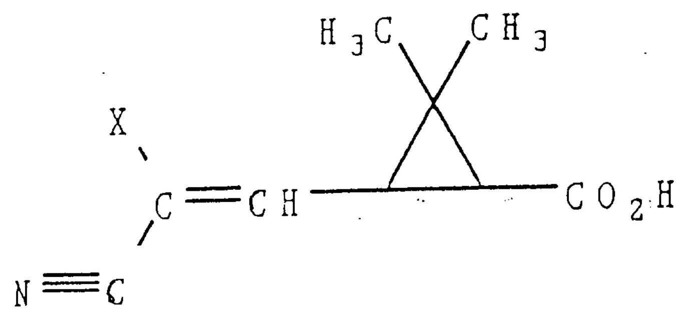

(II)

in der X seine vorstehende Bedeutung beibehält, oder ein Derivat dieser Säure, der Einwirkung eines Alkoholes der Formel (III)

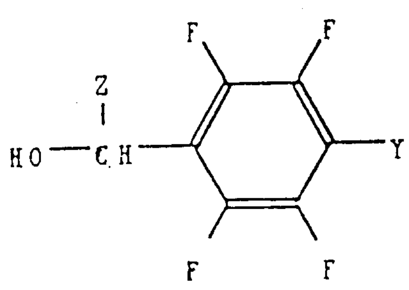

(III)

in der Y und Z ihre Bedeutungen wie in Anspruch 1 angegeben beibehalten, oder eines funktionellen Derivates dieses Alkoholes unterzieht, um die entsprechende Verbindung der Formel (I) zu erhalten.

**12.** Pestizide Zusammensetzungen, die als Wirkstoff mindestens eine Verbindung der Formel ($I_A$) wie in irgendeinem der Ansprüche 1 bis 9 definiert, umfassen.

**13.** Pestizide Zusammensetzungen, die als Wirkstoff mindestens die Verbindung der Formel ($I_A$) wie in Anspruch 10 definiert, umfassen.

**14.** Insektizide Zusammensetzungen, die als Wirkstoff mindestens eine Verbindung der Formel ($I_A$) wie in irgendeinem der Ansprüche 1 bis 10 definiert, umfassen.

**15.** Pestizide Zusammensetzungen wie in Anspruch 12 oder 13 definiert, dadurch gekennzeichnet, daß sie zur Bekämpfung von DIABROTICA oder den anderen Insekten des Bodens vorgesehen sind.